(19) Europäisches Patentamt
European Patent Office
Office européen des brevets

(11) **EP 4 324 466 A1**

(12) **EUROPEAN PATENT APPLICATION**
published in accordance with Art. 153(4) EPC

(43) Date of publication:
**21.02.2024   Bulletin 2024/08**

(51) International Patent Classification (IPC):
*A61K 31/7048* (2006.01)    *A61K 31/352* (2006.01)
*A61K 31/353* (2006.01)    *A61K 36/48* (2006.01)
*A61K 36/488* (2006.01)    *A61K 36/489* (2006.01)
*A61K 36/06* (2006.01)    *A61P 17/14* (2006.01)
*A61K 8/49* (2006.01)    *A23L 33/105* (2016.01)

(21) Application number: 22788282.6

(22) Date of filing: 22.03.2022

(52) Cooperative Patent Classification (CPC):
**A23L 33/105; A61K 8/49; A61K 31/352;**
**A61K 31/353; A61K 31/7048; A61K 36/06;**
**A61K 36/48; A61K 36/488; A61K 36/489;**
**A61P 17/14**

(86) International application number:
**PCT/KR2022/003976**

(87) International publication number:
**WO 2022/220425 (20.10.2022 Gazette 2022/42)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **13.04.2021   KR 20210048075**
**01.07.2021   KR 20210086701**

(71) Applicants:
• **Ben's Lab Co., Ltd.**
**Anyang-si, Gyeonggi-do 14056 (KR)**
• **Jeong, Jong Moon**
**Yongin-si, Gyeonggi-do 16809 (KR)**

• **Lee, Seung Sook**
**Yongin-si, Gyeonggi-do 16809 (KR)**

(72) Inventors:
• **SHIN, Jin A**
**Yongin-si, Gyeonggi-do 16938 (KR)**
• **LI, Hua**
**Hwaseong-si, Gyeonggi-do 18301 (KR)**
• **LEE, Min Jee**
**Suwon-si, Gyeonggi-do 16545 (KR)**
• **JEONG, Jong Moon**
**Yongin-si, Gyeonggi-do 16809 (KR)**
• **LEE, Seung Sook**
**Yongin-si, Gyeonggi-do 16809 (KR)**

(74) Representative: **M. Zardi & Co S.A.**
**Via G. B. Pioda, 6**
**6900 Lugano (CH)**

(54) **PHARMACEUTICAL COMPOSITION FOR HAIR LOSS REDUCTION OR TREATMENT THROUGH INFLAMMATION REDUCTION OF HUMAN DERMAL PAPILLA CELLS AND NEIGHBORING CELLS AND INHIBITION OF 5A-REDUCTASE EXPRESSION**

(57)    The present invention relates to a pharmaceutical composition for alleviating or treating hair loss through the inflammation amelioration of human dermal papilla cells and peripheral cells and the inhibition of 5α-reductase expression, the pharmaceutical composition comprising genistein and kaempferol as active ingredients.

Processed by Luminess, 75001 PARIS (FR)

**(Cont. next page)**

EP 4 324 466 A1

[FIG. 7]

Hair growth pattern for each group

**Description**

**[TECHNICAL FIELD]**

The present invention relates to a pharmaceutical composition for alleviating or treating hair loss, and more particularly to a pharmaceutical composition for alleviating or treating hair loss through the inflammation amelioration of human dermal papilla cells and peripheral cells and the inhibition of 5α-reductase expression.

**[BACKGROUND ART]**

Hair loss refers to a condition in which hair does not exist in areas where hair should normally exist, and generally means loss of terminal hair (thick and black hair) on the scalp. Unlike a soft hair, which is colorless and thin in thickness, the terminal hair can cause cosmetic problems if it falls out. In the case of Korean people, whose hair density is lower than that of Western people, they have approximately 100,000 hairs. Losing about 50 to 80 hairs per a day is considered as a normal phenomenon. Therefore, if the number of hairs that fall out when waking up or washing the hair exceeds 100, it is highly likely that it is due to a pathological cause.

Hair loss may be clinically divided into two types: scarring hair loss (scarring alopecia) and non-scarring hair loss (nonscarring alopecia). In scarring alopecia, hair follicles are destroyed so that hair does not regenerate, whereas in nonscarring alopecia, hair follicles are maintained so that hair can regrow after symptomatic areas disappear. Scarring alopecia in which scars are formed may include hair loss caused by Lupus erythematosus, toxic folliculitis, lichen planus pilaris, hair loss caused by burns and trauma, and the like, and nonscarring alopecia in which scars are not formed may include hereditary androgenic hair loss (baldness), alopecia areata, tinea capitis due to fungal infections, telogen effluvium, trichotillomania, hair production disorders, and the like.

The most frequent forms of alopecia are male pattern alopecia (androgenic), female pattern alopecia and Alopecia areata, all of which are nonscarring alopecia that does not produce scars.

The causes of hair loss are various.

In the occurrence of male pattern alopecia, genetic causes and androgens, which are male hormones, are considered to be important factors. Even in female pattern alopecia, some are estimated to occur through the same path as male pattern alopecia, but there are differences in the clinical aspect. Alopecia areata is generally considered an autoimmune disease caused by extreme stress. Telogen effluvium is a type of temporary hair loss caused by severe physical and psychological stress such as endocrine disorders, nutritional deficiencies, drug use, childbirth, fever, and surgery, in which some of hairs do not complete the growth period and transitions to the telogen state and finally falls out.

In male pattern alopecia, hair becomes gradually thin and hair loss progresses from the 20s or 30s in people with a family history of baldness. While the borderline between the forehead and hair is pushed backward, the forehead widens in an M-shape on both sides of the temporal region, and hair loss gradually progresses to the top of the head.

Female pattern alopecia is characterized by the maintenance of hair lines on the forehead, finer hair in the central part of the head, and less hair while maintaining the hairline on the forehead compared to male pattern alopecia. The degree of hair loss is weak, and thus, it is rare for the forehead to peel off and become complete baldness as in male pattern alopecia.

Alopecia areata is characterized by the occurrence of coin-shaped, circular or oval bald spots (hair disappears and looks like spots) of varying sizes. It occurs mainly on the head, and rarely occurs on the beard, eyebrows, and eyelashes. As the symptomatic area enlarges, a large bald spots may form. If the whole of the hair falls out, it may be classified as a whole head alopecia, and if the whole body hair falls out, it may be classified as whole body alopecia. In telogen effluvium, hair loss begins 2 to 4 months after the occurrence of the causative stimulus, and the hair decreases overall. Once the causative stimulus is removed, hair loss will decrease as the telogen hair returns to normal over several months. A condition where hair does not exist in areas where hair should normally exist, generally loss of terminal hair (thick and dark hair) on the scalp, and the most common types of alopecia, are alopecia areata and baldness, and none of them cause scarring.

For the treatment of male pattern alopecia and female pattern alopecia, toner or lotion type such as minoxidil, oral drugs such as finasteride and dutasteride, and hair transplantation are used, and for the treatment of alopecia areata, topical steroid preparations, systemic steroid preparations, immunotherapy, and the like are used.

Although minoxidil is used in a state where its mechanism of action is clearly identified, it is generally presumed that it improves blood circulation in the scalp and thus facilitates and improves the supply of nutrients and oxygen to the cells that make up the hair root. Hair regrows 4 to 6 months after the start of use, and when treatment is discontinued, the effect disappears again over 4 to 6 months, which exhibits side effects such as scalp irritation, skin dryness, scaling, pruritus, and redness, allergic contact dermatitis, and photoallergic contact dermatitis.

The principle by which finasteride is used to treat male pattern alopecia (androgenic alopecia) and benign prostatic hyperplasia (BPH) is to reduce the production of the hormone dihydrotestosterone (DHT) that causes the disease. Since DHT shrinks hair follicles, causes male pattern alopecia, and promotes prostate growth, reducing DHT can treat male pattern alopecia and benign prostatic hyperplasia. DHT is produced from testosterone by $5\alpha$-reductase, wherein finasteride reduces DHT production by inhibiting $5\alpha$-reductase. When used for male pattern alopecia, it can only be used in adult males and not in females. If a pregnant woman is exposed to finasteride, pregnant women or women who can become pregnant exhibits serious side effects to the extent that should avoid contact with drugs containing this ingredient, as it can cause abnormalities in the external reproductive organs of male fetuses. In addition, when administered to men, it exhibits sexual side effects (decreased libido, decreased ejaculation volume, decreased erectile stiffness, etc.) and side effects such as depression.

Dutasteride exhibits stronger pharmacological effects than finasteride, but it is also used to treat benign prostatic hyperplasia and androgenetic alopecia as a drug that inhibits the production of male hormone DHT. Dutasteride is a drug of the same series as finasteride, is used for the same purpose, and has the same pharmacological action. The difference is that dutasteride blocks both types 1 and 2 of $5\alpha$-reductase, but finasteride blocks only type 2. Therefore, it has been shown that dutasteride decreases DHT concentration more than finasteride. Therefore, when used for male pattern alopecia, it can be used only in adult males, and cannot be used in females. When a pregnant woman is exposed to dutasteride, malformations may be induced in a male fetus, so a pregnant woman or a woman of childbearing potential exhibits serious side effects to the extent that should avoid contact with a drug containing this component. In addition, when administered to men, it also exhibits sexual side effects (decreased libido, decreased ejaculation volume, decreased erectile rigidity, etc.) and side effects such as depression.

However, DHT, which can still cause benign prostatic hyperplasia and hair loss at the same time, promotes cell division in the prostate and enlarges the prostate tissue, whereas there is no clear evidence as to why the tissue forming the hair follicle is conversely atrophied and the hair becomes thin, eventually leading to hair loss.

Therefore, in the present invention, first, human dermal papilla cells, which help the growth and differentiation of keratinocytes and matrix cells that make hair in hair follicles, were cultured, and then testosterone or DHT was added thereto. Human dermal papilla cells were found to increase IL-6, which is particularly a pro-inflammatory cytokine, by about 2 to 4 times under the influence of these androgenic hormones. Moreover, it has been found that when not only DHT but also testosterone is exposed to human dermal papilla cells for a long period of time, a large amount of IL-6 is produced and released from human dermal papilla cells like DHT. Originally, IL-6 is a type of pro-inflammatory cytokine that is mainly produced and secreted by macrophages in blood or tissue in response to external invasion and causes the strongest inflammatory response. Therefore, in the present invention, it was judged that androgenetic alopecia, alopecia areata, and female pattern alopecia also cause inflammation under the influence of IL-6 or TNF-$\alpha$, which is an inflammation-inducing factor that is overproduced and secreted in dermal papilla cells which play the most important role in the production of hair in the hair follicle, and macrophages present in blood or other peripheral tissues, thereby gradually exhausting peripheral cells including hair follicles, and eventually leading to hair loss.

For this purpose, in the present invention, DHT or testosterone was first added to dermal papilla cells to search for a substance that effectively inhibits IL-6 production and secretion under conditions in which IL-6 is produced and secreted as much as possible.

Next, a macrophage cell line that can produce and secrete the largest amount of IL-6 in the peripheral tissue to affect hair follicle tissue is used, and a powerful inflammation-inducing substance called lipopolysaccharide (LPS) is added thereto to create conditions to produce and secrete as much IL-6 or TNF-$\alpha$ as possible, and then we have searched for substances that most effectively inhibit the production and secretion of these inflammatory cytokines. Moreover, we have also searched for substances that effectively inhibit the expression of an inflammatory enzyme called COX-2 (cyclooxygenase type 2), which is generally involved in the production of substances that directly induce inflammation, such as prostaglandins, and iNOS (inducible nitric oxide synthase), which is involved in the production of nitric oxide, which is used to induce inflammation for the first time. In addition to this, we also explored and quantitatively tested whether it actually effectively inhibits the production of nitric oxide, which loosens blood vessels to facilitate the recruitment of many inflammatory cells to the site of inflammation.

Further, most of the hair loss treatment agents were developed focused on inhibiting the activity of $5\alpha$-reductase, which catalyzes the conversion of testosterone to DHT, by using a kind of competitive inhibitor function with substances similar in molecular structure to testosterone, such as finasteride, whereas in the present invention, from the beginning, we have searched for a substance that inhibits the expression of this $5\alpha$-reductase.

In addition, under the assumption that inflammatory cells called mast cells induce inflammation in hair follicles, including the scalp, along with allergy-inducing substances such as histamine and leukotriene secreted by external stimuli, eventually leading to hair loss, a search was made for substances that can calm down mast cells and effectively inhibit histamine secretion, or effectively inhibits the expression of 5-LO (Lipoxygenase type 5), a representative inflammatory enzyme involved in the production of leukotriene, which is a potent allergenic substance.

Finally, due to the fact that reactive oxygen, which is abundantly produced by microbial infection, stress, and other environmental substances, causes chronic inflammation, we have searched for substances with excellent antioxidant ability that can scavenge reactive oxygen.

Currently used hair loss therapies largely include a method that directly applies to the scalp like minoxidil, which supplies nutrients and oxygen to hair follicles through improved blood circulation, a method of inhibiting $5\alpha$-reductase, such as finasteride or dutasteride, to prevent the conversion of testosterone to DHT, thereby lowering the concentration of DHT in the blood, and steroids such as triamcinolone, desoximetasone, and betamethasone, and light therapy. However, an increasing number of patients suffer from decreased sexual function or other side effects of drugs such as Propecia using finasteride, and particularly, since its use is very difficult for female alopecia, there is an urgent need to develop hair loss therapeutic agents or functional foods derived from natural substances with no side effects or with very few side effects. In addition, steroids such as corticosteroids cause various side effects in the human body when used for a long period of time, and thus are recommended for use only as short-term prescriptions by doctors. Further, minoxidil, which has relatively few side effects compared to other drugs as a topical preparation, is accompanied by troublesome effects that are expected to be effective only after 4 to 6 months of continuous use. Therefore, in order to solve such overall problems, we have searched for naturally occurring substances that not only fundamentally remove inflammation occurring in peripheral tissues including hair follicles due to stimulation by continuous stimulation of androgen hormones, stress, environmental hormones, contaminants, disease infection, etc., but also have complex functions such as inhibiting the expression of $5\alpha$-reductase, which catalyzes the production of DHT, and effectively scavenges active oxygen, while causing no or very little harm to the human body even when used for a long period of time. Thus, there is an urgent need to develop such substances into hair loss therapeutic agents, functional foods, cosmetic products, and the like.

## [DETAILED DESCRIPTION OF THE INVENTION]

### [Technical Problem]

The present inventors have conducted intensive research to discover naturally occurring compounds that can prevent hair loss or promote hair growth or hair restoration without side effects. As a result of searching for about 30 types of naturally derived substances, the inventors have found that a composition containing genistein and kaempferol as active ingredients effectively promotes hair growth and restoration and prevents hair loss, and completed the present invention.

Therefore, an object of the present invention is to provide a pharmaceutical composition for alleviating or treating hair loss through the inflammation amelioration (including allergy) of human dermal papilla cells and peripheral cells and the inhibition of $5\alpha$-reductase expression, the pharmaceutical composition comprising genistein and kaempferol as active ingredients; and a pharmaceutical product (quasi-drug), a cosmetic composition or a functional food composition for alleviating or treating hair loss.

### [Technical Solution]

According to an exemplary embodiment of the present invention, there is provided a pharmaceutical composition for alleviating or treating hair loss through the inflammation amelioration of human dermal papilla cells and peripheral cells and the inhibition of $5\alpha$-reductase expression, the pharmaceutical composition comprising genistein and kaempferol as active ingredients.

According to another exemplary embodiment of the present invention, there is provided a pharmaceutical product (quasi-drug) for alleviating or treating hair loss through the inflammation amelioration of human dermal papilla cells and peripheral cells and the inhibition of $5\alpha$-reductase expression, the pharmaceutical product (quasi-drug) comprising genistein and kaempferol as active ingredients.

According to yet another exemplary embodiment of the present invention, there is provided a cosmetic composition for alleviating or treating hair loss through the inflammation amelioration of human dermal papilla cells and peripheral cells and the inhibition of $5\alpha$-reductase expression, the cosmetic composition comprising genistein and kaempferol as active ingredients.

According to still yet another exemplary embodiment of the present invention, there is provided a functional food composition for alleviating or treating hair loss through the inflammation amelioration of human dermal papilla cells and peripheral cells and the inhibition of $5\alpha$-reductase expression, the functional food composition comprising genistein and kaempferol as active ingredients.

### [Advantageous Effects]

According to the present invention, there is provided a pharmaceutical composition for alleviating or treating hair loss through the inflammation amelioration of human dermal papilla cells and peripheral cells and the inhibition of $5\alpha$-reductase expression, the pharmaceutical composition comprising genistein and kaempferol as active ingredients.

Specifically, a pharmaceutical composition for alleviating or treating hair loss; a pharmaceutical product (quasi-drug) for alleviating or treating hair loss; a cosmetic composition for alleviating or treating hair loss; and a functional food composition for alleviating or treating hair loss is provided in which each not only comprises natural compounds having little or no side effects even after long-term administration in the chronic disease of hair loss, as an active ingredient, but also is excellent in hair growth and restoration, exhibits stable efficacy, and is effective for alleviating or treating hair loss.

[BRIEF DESCRIPTION OF THE DRAWINGS]

FIG. 1 is an electrophoresis photograph (Western blot) showing the results of measuring the iNOS and COX-2 expression inhibitory effects of epigallocatechin gallate, genistein and kaempferol.

FIG. 2 is a graph showing the results of measuring the nitric oxide (NO) secretion inhibitory effects of epigallocatechin gallate, genistein and kaempferol.

FIG. 3 is a graph showing the results of measuring the IL-6 secretion inhibitory effect produced by testosterone and DHT of epigallocatechin gallate, genistein and kaempferol.

FIG. 4 is an electrophoresis photograph (Western blot) showing the results of measuring the $5\alpha$-reductase type II expression inhibitory effects of epigallocatechin gallate, genistein and kaempferol.

FIG. 5 is an electrophoresis photograph (Western blot) showing the results of measuring the 5-LO expression inhibitory effects of epigallocatechin gallate, genistein and kaempferol.

FIGS. 6 and 7 are diagrams showing the results of visual evaluation of the hair growth rate by photographing the back skin of all mice with a digital camera, just before the start of oral administration, and on day 7, day 12, day 17, day 22, and day 27 after oral administration, of a composition for ameliorating hair loss (KGE complex: 40% by weight of kaempferol, 30% by weight of genistein and 30% by weight of epigallocatechin gallate) as a specific embodiment of the present invention (day 0).

FIG. 8 is a graph showing the results of measuring the length of grown hair, after completion of oral administration test (day 27) of a composition for ameliorating hair loss (KGE complex: 40% by weight of kaempferol, 30% by weight of genistein and 30% by weight of epigallocatechin gallate) to mice as a specific embodiment of the present invention.

FIG. 9 is a graph showing the results of measuring the weight of grown hair, after completion of oral administration test (day 27) of a composition for ameliorating hair loss (KGE complex: 40% by weight of kaempferol, 30% by weight of genistein and 30% by weight of epigallocatechin gallate) as a specific embodiment of the present invention to mice.

FIG. 10 is a graph showing visual evaluation results for each oral administration group.

FIG. 11 is photographs arranged by date of actual hair growth of mice in each oral administration group, taken with a camera.

FIG. 12 is a graph showing the results of measuring the hair length of mice in each oral administration group.

FIG. 13 is a graph showing the results of measuring the total hair weight of mice in each oral administration group.

FIG. 14 is a photograph of hair density before and 8 weeks after oral administration of a composition for ameliorating hair loss (KGE complex: 40% by weight of kaempferol, 30% by weight of genistein and 30% by weight of epigallocatechin gallate) as one specific example of the present invention to humans.

FIG. 15 is a photograph of hair thickness before and 8 weeks after oral administration of a composition for ameliorating hair loss (KGE complex: 40% by weight of kaempferol, 30% by weight of genistein and 30% by weight of epigallocatechin gallate) as one specific example of the present invention to humans.

**[DETAILED DESCRIPTION OF THE EMBODIMENTS]**

In exemplary embodiments, provided herein is a pharmaceutical composition for alleviating or treating hair loss, comprising genistein and kaempferol as active ingredients.

Also provided herein is a pharmaceutical product (quasi-drug) for alleviating or treating hair loss, comprising genistein and kaempferol as active ingredients.

Further provided herein is a cosmetic composition for alleviating or treating hair loss, comprising genistein and kaempferol as active ingredients.

Further provided herein is a functional food composition for alleviating or treating hair loss, comprising genistein and kaempferol as active ingredients.

Further provided herein is a composition which alleviates inflammation of hair follicles, peripheral tissues and cells by using 5 to 950 mg/day of genistein as a sole active ingredient to thereby treat or ameliorate hair loss.

[Embodiments of the Invention]

Now, specific embodiments of the present invention will be described in detail with reference to the accompanying drawings.

A pharmaceutical composition for alleviating or treating hair loss according to an embodiment of the present invention comprises genistein and kaempferol as active ingredients. The pharmaceutical composition for alleviating or treating hair loss according to an embodiment of the present invention exhibits the effects of alleviating or treating hair loss through the inflammation amelioration of human dermal papilla cells and peripheral cells and the inhibition of $5\alpha$-reductase expression.

Genistein is a BCS class II isoflavone commercially available from a number of sources including LC Laboratories (Woburn, MA), which is a plant-based estrogen analogue having strong antioxidant, anti-inflammatory, anti-cancer effects, and promoting normal cell replication with hormonal and cell regenerative effects as an estrogen analogue. Recently, clinical results have been reported as a substance that inhibits photoaging. Cellular targets for genistein and signaling pathways regulated by genistein have been identified, and those associated with cancer include targets and pathways important for cell growth and division, apoptosis, angiogenesis, tumor invasion and metastasis. In addition to the inherent anti-tumor effects of genistein itself, studies also showed that genistein enhances or potentiates the anti-tumor effects of several clinically used chemotherapeutic agents both in vitro in human cancer cell lines and in vivo in animal models of cancer. Genistein, a type of polyphenol, can be isolated and extracted from, for example, lupine, fava beans, soy beans, kudzu root or Sophora japonica (pagoda tree) fruits, but is not limited thereto, and it can also be synthesized. In the present invention, genistein is used as it is or in the form of a pharmaceutically acceptable salt, and is not particularly limited as long as it is, for example, sodium, potassium, magnesium, N-methylglucamine (meglumine), calcium, L-lysine, N-ethylglucamine (egglumine), and diethylamine salts of crystalline genistein, and further a pharmaceutically acceptable non-toxic salt including crystalline hydrates of genistein, such as monohydrate and dihydrate. The genistein can be taken in an amount ranging from 1 to 950 mg/day.

Kaempferol is one of the typical components of flavonol, which is one of the flavonoids, and is widely distributed in flowers or leaves of plants. More than 100 types of flavonols are already known, and three types of kaempferol, quercetin, and myricetin are known to exist in the largest amount. In particular, kaempferol is a substance excellent in physiological activities such as antioxidant and anti-inflammatory properties, and its various effects have been studied and applied to various fields. In the present invention, kaempferol can be used as it is or in the form of a pharmaceutically acceptable salt. The salt used in the present invention is not particularly limited as long as it is a pharmaceutically acceptable non-toxic salt. For example, it can be used in the form of a salt, such as hydrochloric acid, sulfuric acid, nitric acid, phosphoric acid, hydrofluoric acid, hydrobromic acid, formic acid, acetic acid, tartaric acid, lactic acid, citric acid, fumaric acid, maleic acid, succinic acid, methane sulfonic acid, benzene sulfonic acid, toluene sulfonic acid, naphthalene sulfonic acid, or the like. In addition, kaempferol can be used by extracting and concentrating it from natural plants such as capers, saffron, kale, ginger, Chinese cabbage, and persimmon leaves, and is not necessarily limited thereto. The kaempferol can be taken in an amount ranging from 5 to 9,500 mg/day.

The pharmaceutical composition for alleviating or treating hair loss according to an exemplary embodiment of the present invention may contain genistein in an amount ranging from 5 to 95% by weight and kaempferol in an amount ranging from 5 to 95%, preferably genistein in an amount ranging from 20 to 60% by weight and kaempferol in an amount ranging from 40 to 80%.

The pharmaceutical composition for alleviating or treating hair loss according to the present invention may further comprise epigallocatechin gallate (EGCG).

Epigallocatechin gallate (EGCG), also called gallate-3-epigallocatechin, is a component that is a type of polyphenol, an extract of green tea leaves. Green tea extract contains about 3 to 4% of caffeine on a weight basis, and contains 0 to 18% of polyphenolic components known as catechins, wherein caffeine and catechin are known as the main components of green tea. The catechin component is composed of catechin (C), epicatechin (EC), epigallocatechin gallate (EGCG), epigallocatechin (EGC), epicatechin gallate (ECG), and the like. Among them, EGCG accounts for more than half of all catechins. It has been reported that epigallocatechin gallate has a strong antioxidant activity, also has antiviral ability against influenza virus influenza, inhibits the growth of prostate cancer, and has a therapeutic effect on leukemia. In addition, it has been reported that it is effective in delaying or preventing diabetes, hyperlipidemia, and arteriosclerosis which are major adult diseases.

The pharmaceutical composition for alleviating or treating hair loss according to the present invention may contain 20 to 60% by weight of genistein, 40 to 80% by weight of kaempferol and the balance of epigallocatechin gallate, and preferably, it may contain 25 to 45% by weight of genistein, 35 to 55% by weight of kaempferol and the balance of epigallocatechin gallate.

The pharmaceutical composition for alleviating or treating hair loss according to the present invention may further comprise a yeast extract.

Yeast extract means an aqueous yeast extract, and the yeast is not limited to a particular type, and examples thereof include *Saccharomyces cerevisiae, Saccharomyces ellipsoideus, Saccharomyces sake, Saccharomyces formosensis, Saccharomyces coreanus, Saccharomyces boulardii,* and the like. *Saccharomyces cerevisiae* is preferred. Yeast contains various amino acids such as arginine, glutamic acid, isoleucine, leucine, and valine, and thus, plays a role in supplementing protein deficiency in skin or hair. In addition, yeast is known to contain various vitamins such as vitamin B1 (thiamine), vitamin B2 (riboflavin), pantothenic acid, niacin, biotin, and the like. Due to such properties, it is expected to exhibit various effects on human health. In the present invention, such an aqueous yeast extract is used, and the yeast extract that can be used at this time may include a yeast extract obtained by dissolving yeast in water, separating the dissolved portion and the undissolved portion by filtration, separating the dissolved portion from the undissolved portion, separately taking the dissolved portion and concentrating it, and filtering the concentrate, or a yeast extract obtained by crushing by a crushing method using heat or salt or both at the same time to obtain an extract, precipitating this with 50 to 70% ethanol, redissolving the precipitate, and removing the insoluble portion, but is not limited thereto. Yeast extract may be included in an amount ranging from 10 to 50 parts by weight based on 100 parts by weight of the pharmaceutical composition for alleviating or treating hair loss according to the present invention.

The pharmaceutical composition for alleviating or treating hair loss according to the present invention as described above can alleviate and/or treat hair loss and promote hair growth and hair restoration through the inflammation amelioration of human dermal papilla cells and peripheral cells and the inhibition of 5α-reductase expression.

The pharmaceutical composition for alleviating or treating hair loss according to the present invention may be formulated into various oral and parenteral dosage forms, and when formulated, it may be prepared using diluents or excipients such as commonly used fillers, extenders, binders, wetting agents, disintegrants, and surfactants. The dosage of such a composition varies depending on the patient's weight, age, sex, health condition, diet, administration time, administration method, excretion rate and severity of the disease, and the daily dosage may be about 0.0001 to 1 g/kg of body weight based on the amount of the composition, and may be administered 1 to 6 times a day. It can be formulated as a composition for oral administration containing this composition as an active ingredient. That is, in addition to the above composition as an active ingredient, it may include one or more fillers such as microcrystalline cellulose, lactose, sugars, starches, modified starches, mannitol, sorbitol and other polyols, dextrin, dextran and maltodextrin, calcium carbonate, calcium phosphate and/or hydrogen phosphate, and sulfuric acid; one or more binders such as lactose, starch, modified starch, dextrin, dextran and maltodextrin, microcrystalline cellulose, sugars, polyethylene glycol, hydroxypropyl methylcellulose, ethylcellulose, hydroxyethylcellulose, methylcellulose, carboxymethylcellulose, sodium carboxymethylcellulose, gelatin, gum acacia, tragacanth, polyvinylpyrrolidone, magnesium aluminum silicate, and the like; one or more disintegrating agents such as bridged sodium carboxymethylcellulose, bridged polyvinylpyrrolidine, bridged carboxymethyl starch, starch and microcrystalline cellulose, magnesium aluminum silicate, potassium polyacrylate, and the like; one or more different glidants such as magnesium, calcium and zinc stearate, calcium behenate, sodium stearyl fumarate, talc, magnesium trisilicate, stearic acid, palmitic acid, carnauba wax, silicon dioxide, and the like; one or more buffering agents such as sodium citrate, dibasic sodium phosphate, calcium carbonate, hydrogen phosphate, phosphate, sulfate, magnesium carbonate, potassium citrate, potassium sorbate, sodium ascorbate, benzoate, hydrogen carbonate, hydrogen phosphate, and the like; or one or more basic substances such as MgO, MgOH, NaOH, $Ca(OH)_2$, tromethamine, aluminum magnesium silicate, lauryl sulfate, and the like. If necessary, the dosage form may contain surfactants and other useful ingredients for solid pharmaceutical dosage forms, such as coloring agents, lakes, aromas, and adsorbents. As the surfactant, the following may be used: ionic surfactants such as sodium lauryl sulfate, poloxamers (polyoxyethylene and polyoxypropylene polymers) that are different from each other, natural or synthetic lecithins, sorbitan fatty acid ester (e.g., Span® available from Atlas Chemie), polyoxyethylene sorbitan fatty acid esters (e.g., Tween® available from Atlas Chemie), polyoxyethylene hydrogenated castor oil (e.g., Cremophor® available from BASF), nonionic surfactants such as polyoxyethylene stearate (e.g., Myrj® available from Atlas Chemie) or cationic surfactants such as cetyl pyridine chloride or mixtures of the above-mentioned surfactants. The pharmaceutical dosage form of the present invention can be prepared in solid pharmaceutical dosage form such as powder, granule, tablet or capsule, or in liquid pharmaceutical dosage form, preferably in encapsulated form. A solid pharmaceutical dosage form according to the invention can be prepared as follows.

The active ingredients, excipients, binders, alkalizing or buffering substances, disintegrants and optionally, surfactants and mixtures of other ingredients useful in solid pharmaceutical dosage forms are homogenized using a suitable mixer. The mixture is pressed using a suitable pressing machine or slugged on a slugging machine, and the pressed material or slug is grinded and/or sieved. Excipients, disintegrants, buffer substances, glidants, lubricants and other ingredients useful for tablets or capsules are added thereto and re-homogenized. The resulting mixture is compressed into tablets or filled into soft or hard capsules.

The active ingredients, excipients, binders, alkalizing or buffering substances, disintegrants and optionally, surfactants and mixtures of other ingredients useful in solid pharmaceutical dosage forms are homogenized using a suitable mixer. The glidants and lubricants are added thereto and re-homogenized. The resulting mixture is compressed into tablets or filled into soft or hard capsules.

The active ingredients, excipients, binders, alkalizing or buffering substances, disintegrants and optionally, surfactants and mixtures of other ingredients useful in solid pharmaceutical dosage forms are homogenized using a suitable mixer, and granulated with an appropriate solvent such as water, ethanol, methanol, isopropyl alcohol, n-butyl alcohol, acetone, diethyl ether, ethyl acetate, isopropyl acetate, methyl acetate, ethyl acetate, isopropyl acetate, methyl acetate, dichloromethane, chloroform, a mixture of solvents such as ethanol and acetone, methanol and acetone, dichloromethane and methanol, and mixtures thereof. The resulting granules are dried in suitable dryers such as standard tray dryers, fluid bed dryers, vacuum and electromagnetic wave dryers. Glidants, disintegrants, alkalinizing or buffering substances, glidants and lubricants are added to the dried granules, and optionally other ingredients useful in solid pharmaceutical dosage forms. The resulting mixture can be re-homogenized and compressed into tablets or filled into soft or hard capsules. Optionally, the tablets can be further film-coated.

According to another exemplary embodiment of the present invention, there is provided a pharmaceutical product (quasi-drug) for alleviating or treating hair loss through the inflammation amelioration of human dermal papilla cells and peripheral cells and the inhibition of $5\alpha$-reductase expression, the pharmaceutical product (quasi-drug) comprising genistein and kaempferol as active ingredients. When used as a pharmaceutical product (quasi-drug) composition for alleviating or treating hair loss according to the present invention, the above composition for alleviating or treating hair loss may be used as it is or in combination with other pharmaceutical product (non-drug) ingredients, and may be used as appropriate according to conventional methods. The mixing amount of the active ingredients may be appropriately determined according to the purpose of use (prevention, health or therapeutic treatment). According to the present invention, the pharmaceutical product (quasi-drug) composition of the present invention may be a disinfectant cleaner, shower foam, gargle, wet tissue, detergent soap, hand wash, humidifier filler, mask, ointment or filter filler.

According to yet another exemplary embodiment of the present invention, there is provided a cosmetic composition for mitigating or treating hair loss through the inflammation amelioration of human dermal papilla cells and peripheral cells and the inhibition of $5\alpha$-reductase expression, the cosmetic composition comprising genistein and kaempferol as active ingredients. When used as a cosmetic composition for mitigating or treating hair loss according to the present invention, the cosmetic composition may include, in addition to the above composition for mitigating or treating hair loss, ingredients commonly used in cosmetic compositions. For example, it may include conventional adjuvants such as antioxidants, stabilizers, solubilizers, vitamins, pigments and flavors, and carriers. The cosmetic composition of the present invention may be prepared in any dosage form conventionally prepared in the art, and for example, it may be formulated into solutions, suspensions, emulsions, pastes, gels, creams, lotions, powders, shampoos, soaps, surfactant-containing cleansers, oils, scalp ampoules or toners, powder foundations, emulsion foundations, wax foundations, sprays, and the like, but is not limited thereto.

When the dosage form of the present invention is a paste, cream or gel, animal oil, vegetable oil, wax, paraffin, starch, tragacanth, cellulose derivatives, polyethylene glycol, silicone, bentonite, silica, talc or zinc oxide may be used as the carrier component.

When the dosage form of the present invention is a powder or spray, lactose, talc, silica, aluminum hydroxide, calcium silicate or polyamide powder can be used as a carrier component, and especially when it is a spray, it may further contain propellants such as chlorofluorohydrocarbons, propane/butane or dimethyl ether.

When the dosage form of the present invention is a solution or emulsion, a solvent, a solubilizing agent or an emulsifying agent is used as a carrier component, and examples thereof include water, ethanol, isopropanol, ethyl carbonate, ethyl acetate, benzyl alcohol, benzyl benzoate, propylene glycol, 1,3-butylene glycol oil, glycerol aliphatic acid ester, polyethylene glycol or fatty acid esters of sorbitan.

When the dosage form of the present invention is a suspension, liquid diluents such as water, ethanol or propylene glycol, suspending agents such as ethoxylated isostearyl alcohols, polyoxyethylene sorbitol esters and polyoxyethylene sorbitan esters, microcrystalline cellulose, aluminum methahydroxide, bentonite, aga or tragacanth and the like can be used as a carrier component.

When the dosage form of the present invention is a surfactant-containing cleanser, aliphatic alcohol sulfate, aliphatic alcohol ether sulfate, sulfosuccinic acid monoester, isethionate, imidazolinium derivatives, methyl taurates, sarcosinates, fatty acid amide ether sulfates, alkylamidobetaines, fatty alcohols, fatty acid glycerides, fatty acid diethanolamides, vegetable oils, lanolin derivatives or ethoxylated glycerol fatty acid esters, and the like can be used as a carrier component.

According to yet another exemplary embodiment of the present invention, there is provided a functional food composition for alleviating or treating hair loss through the inflammation amelioration of human dermal papilla cells and peripheral cells and the inhibition of $5\alpha$-reductase expression, the functional food composition comprising genistein and kaempferol as active ingredients.

When the composition of the present invention is prepared as a functional food composition or a food composition, it includes functional foods or ingredients that are usually added during food production, in addition to the composition for alleviating or treating hair loss as described above as active ingredients, and examples thereof may include proteins, carbohydrates, fats, nutrients, seasonings and flavoring agents. Examples of the aforementioned carbohydrates include monosaccharides such as glucose, fructose and the like, disaccharides such as maltose, sucrose, oligosaccharides and the like, and polysaccharides, for example, common sugars such as dextrins, cyclodextrins, and the like, and sugar alcohols such as xylitol, sorbitol, and erythritol. As the flavoring agent, natural flavoring agents (thaumatin, stevia extract (e.g., rebaudioside A, glycyrrhizin, etc.)) and synthetic flavoring agents (saccharin, aspartame, etc.) can be used. When the functional food composition of the present invention is produced as a drink, it may further include citric acid, liquid fructose, sugar, glucose, acetic acid, malic acid, fruit juice, eucommia extract, jujube extract or licorice extract, in addition to the composition for alleviating or treating hair loss as described above.

Since the pharmaceutical product (quasi-drug), the cosmetic composition, and the functional food composition of the present invention share active ingredients and uses with the above-described pharmaceutical composition, descriptions of common contents in relation to the pharmaceutical composition are omitted in order to avoid undue complexity of the present specification.

Preferred Examples and Comparative Examples of the present invention will be described below.

The following examples are intended to illustrate the present invention and should not be construed as limiting the scope of the present invention.

Example 1: Anti-inflammatory Test

1. Expression inhibition of inducible nitric oxide synthase (iNOS) and cyclooxygenase-2 (COX-2)

Mouse macrophage RAW 264.7 was dispensed into a 6-well plate at a concentration of $6\times10^5$ cells/well (number of cells per well), and cultured in an incubator at 37°C under 5% $CO_2$ using a DMEM Dulbecco's Modified Eagle's Medium (Sigma-Aldrich, Cat. No. D7777) for 24 hours, and then samples were added according to the concentrations.

Thirty minutes after the addition of the sample, lipopolysaccharide (LPS, 100 ng/ml) was added, and further cultured for 20 hours. After that, the medium was removed, washed with phosphate buffered saline (PBS) to collect the cells. In order to extract the protein, a lysis buffer for radioimmunoprecipitation assay (RIPA; sodium chloride 150 mM, sodium dodecyl sulfate (SDS) 0.1%, tris-hydrochloric acid (Tris-HCl: pH 7.5) 50 mM, ethylenediaminetetraacetic acid (EDTA) 2 mM, sodium deoxycholate 0.5%, Triton X-100 1%) was added and reacted, and then centrifuged at 10,000 rpm and 4°C for 15 minutes to obtain the supernatant. The absorbance of the supernatant was then measured at 595 nm to determine the protein concentration of each sample. Then, the cell protein sample was mixed with sodium dodecyl sulfate (SDS) and a stain, and subjected to electrophoresis on a 12% SDS polyacrylamide gel. The separated protein was transferred to a polyvinylidene fluoride (PVDF) membrane, blocked with 10% skim milk at room temperature for 1 hour, then attached with a primary antibody and a secondary antibody, and then color-developed with an enhanced chemiluminescent (ECL) solution to measure the expression of iNOS and COX-2.

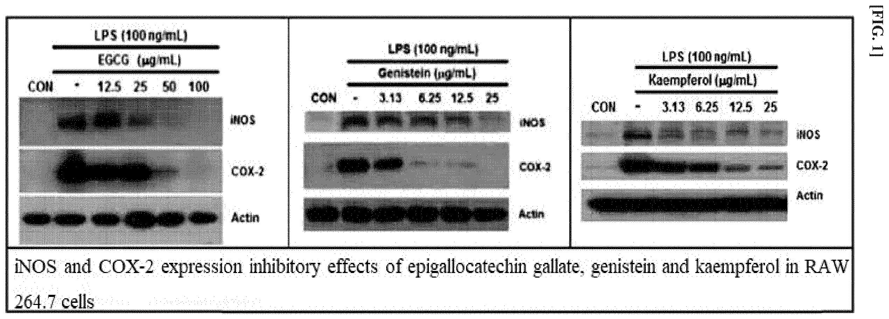

The results are shown in FIG. 1, and it was confirmed that kaempferol inhibited the expression of iNOS to the control (CON) levels at a concentration of 25 $\mu$g/ml, and genistein mostly inhibited the expression of COX-2 at the same concentration (25 $\mu$g/ml).

2. Inhibitory effect on nitric oxide production in macrophages

RAW 264.7 cells, which is a mouse macrophage cell line, were dispensed in a 24-well plate at a concentration of $2\times10^5$ cells/well, and cultured in an incubator at 37°C under 5% $CO_2$ for 24 hours. Epigallocatechin gallate (EGCG), genistein, and kaempferol, which were samples dissolved in DMSO, were added thereto according to the concentrations. At this time, the final concentration of DMSO was adjusted to 0.1%. After 30 minutes, LPS (lipopolysaccharide), which is an inflammation inducing substance, was added at a concentration of 1 $\mu$g/ml. After that, these were cultured in an incubator at 37°C under 5% $CO_2$ for 24 hours.

To quantify nitric oxide, 50 $\mu\ell$ of Griess reagent A (5% phosphoric acid, 1% sulfanilamide) was added to 50

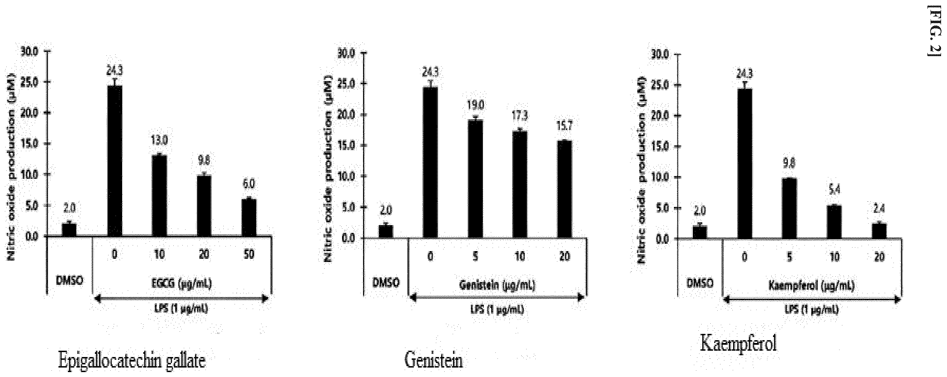

$\mu\ell$ of the cell culture medium, and then the mixture was allowed to stand in a dark place for 5 minutes. 50 $\mu\ell$ of Griess reagent B (0.1% N-(1-naphthyl)ethylenediamine dihydrochloride) was further added thereto, and allowed to react in a dark place for 5 minutes. The absorbance of this reactant was measured at 540 nm, which was compared with the standard curve to calculate the final concentration of nitrogen monoxide. A standard curve was obtained by reacting in the same manner as above using sodium nitrite instead of the cell culture medium and then measuring the absorbance. The results are shown in FIG. 2, wherein kaempferol inhibited almost to the control level at a concentration of 20 $\mu$g/ml, EGCG was the next most inhibitory, and genistein slightly decreased the inhibitory power.

3. Inhibiting the secretion of interleukin-6 (IL-6) and tumor necrosis factor-$\alpha$ (TNF-$\alpha$) secretion in macrophages

Mouse macrophage RAW 264.7 was dispensed into a 24-well plate at a concentration of $2\times10^5$ cells/well, and cultured in an incubator at 37°C under 5% $CO_2$ using a DMEM medium for 24 hours, and then samples were added according to the concentrations. Thirty minutes after addition of the sample, LPS (100 ng/ml) was added and cultured at 37°C for 20 hours to collect the culture medium. The secreted IL-6 and TNF-$\alpha$ were respectively quantified using Mouse IL-6 ELISA kit (Invitrogen, Thermo Scientific) and Mouse TNF-$\alpha$ ELISA kit (Invitrogen, Thermo Scientific).
The results are shown in Table 1 below, and it was confirmed that in macrophages, kaempferol effectively inhibited both IL-6 and TNF-$\alpha$, showing the highest inhibitory effect among epigallocatechin gallate (EGCG), genistein, and kaempferol.

[Table 1] Inhibitory effect of IL-6 and TNF-$\alpha$ release in mouse macrophages (RAW 264.7 cells)

|  | Epigallocatechin gallate (EGCG) | Genistein | Kaempferol |
|---|---|---|---|
| IL-6 ($IC_{50}$) | 98.99 ± 10.87 $\mu$g/m$\ell$ | 8.92 ± 0.08 $\mu$g/m$\ell$ | 5.93 ± 0.07 $\mu$g/m$\ell$ |
| TNF-$\alpha$ ($IC_{50}$) | 83.68 ± 3.45 $\mu$g/m$\ell$ | 14.99 ± 0.11 $\mu$g/m$\ell$ | 13.58 ± 0.72 $\mu$g/m$\ell$ |

4. Inhibition of IL-6 secretion in human follicle dermal papilla cells (HFDPC)

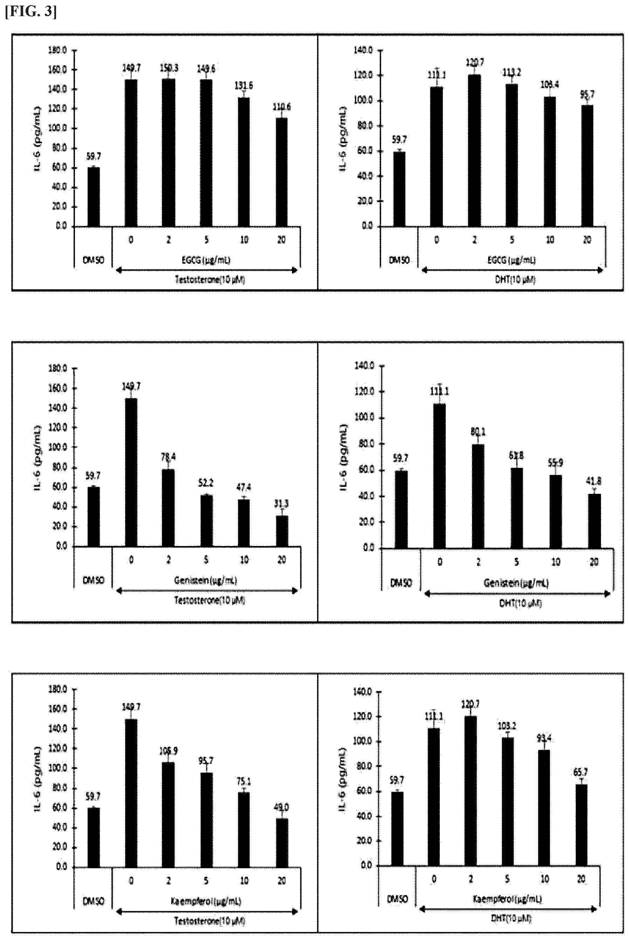

Human follicle dermal papilla cells (HFDPC) were dispensed into a 12-well plate at a concentration of $1\times10^5$ cells/well, and cultured for 24 hours. After that, the medium was replaced with Dulbecco's modified eagle's medium (DMEM) containing no fetal bovine serum (FBS), and subjected to starvation for 24 hours, and then samples were added according to the concentrations (2, 5, 10, 20 $\mu$g/ml). One hour after addition of the sample, testosterone (TST, 10 $\mu$M) or dihydrotestosterone (DHT, 10 $\mu$M) was added, and cultured for 72 hours. Then, the medium was collected, and IL-6 secreted in the medium was quantified using a Human IL-6 ELISA kit (Invitrogen, Thermo Scientific).
The results are shown in FIG. 3. It was confirmed that genistein at a concentration of 5 $\mu$g/ml and kaempferol at a concentration of 10 $\mu$g/ml almost completely inhibited the release of IL-6 by DHT or TST.

Example 2: Inhibition of 5$\alpha$-reductase type II expression in human dermal papilla cells (HFDPC)

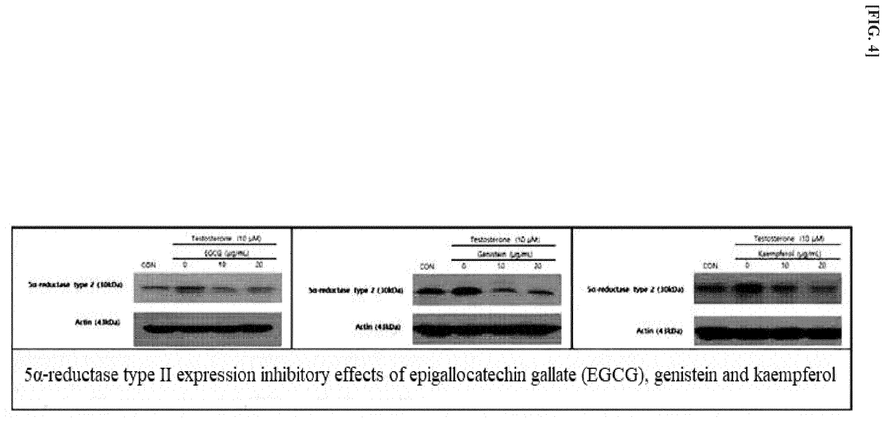

Human dermal papilla cells (HFDPC) were dispensed in a 6-well plate at a concentration of $3\times10^5$ cells/well, and cultured in a DMEM medium for 24 hours, and then the medium was replaced with a DMEM medium containing no FBS, and subjected to starvation for 24 hours, and then samples were added according the concentrations (10, 20 $\mu$g/ml). After 30 minutes, testosterone (10 $\mu$M) was added thereto, and cultured for 72 hours. The medium was then removed, and cells were collected by washing with PBS to extract proteins. To extract a protein, a lysis buffer for radio immunoprecipitation assay (RIPA) (sodium chloride 150 mM, sodium dodecyl sulfate (SDS) 0.1%, tris-hydrochloric acid (Tris-HCl: pH 7.5) 50 mM, ethylenediaminetetraacetic acid (EDTA) 2 mM, sodium deoxycholate 0.5%, Triton X-100 1%) was added and reacted, and then centrifuged at 10,000 rpm and 4°C for 15 minutes to collect the supernatant. Then, the absorbance was measured at 595 nm to determine the protein concentration of each sample. These cell protein samples were mixed with sodium dodecyl sulfate (SDS) and a stain, and subjected to electrophoresis on a 12% SDS polyacrylamide gel. The separated protein was transferred to a polyvinylidene fluoride (PVDF) membrane, blocked at room temperature for 1 hour with 10% skim milk, sequentially attached with a primary antibody that binds to 5$\alpha$-reductase type II and a secondary antibody that binds to the primary antibody, and then color-developed with an enhanced chemiluminescent (ECL) solution to measure the expression of 5$\alpha$-reductase type II.
The results are shown in FIG. 4, and it was confirmed that genistein, kaempferol, and epigallocatechin gallate (EGCG) effectively inhibited the expression of 5$\alpha$-reductase type II in that order.

Example 3: Anti-allergic effect

1. Inhibition of S-lipoxygenase (5-LO) expression

White rat basophilic leukemia cells (RBL-2H3) were dispensed into a 6-well plate at a concentration of $3 \times 10^5$ cells/well, and cultured in an incubator at 37°C under 5% $CO_2$ using a DMEM medium for 24 hours. Samples were added according to the concentrations, cultured for 24 hours, and then Compound 48/80 (1 mg/ml), which is substance that induces an allergic reaction, was added, and cultured for 1 hour. After that, the medium was removed, and washed with PBS to collect the cells, and then proteins were extracted. To extract protein, lysis buffer for radioimmunoprecipitation assay (RIPA; sodium chloride 150 mM, sodium dodecyl sulfate (SDS) 0.1%, tris-hydrochloric acid (Tris-HCl: pH 7.5) 50 mM, ethylenediaminetetraacetic acid (EDTA) 2 mM, sodium deoxycholate 0.5%, Triton X-100 1%) was added and reacted, and then centrifuged at 10,000 rpm and 4°C for 15 minutes to take the supernatant. The absorbance thereof was then measured at 595 nm to determine the protein concentration of each sample. Then, the cell protein sample was mixed with sodium dodecyl sulfate (SDS) and a stain, and subjected to electrophoresis on a 12% SDS polyacrylamide gel. The separated protein was transferred to a polyvinylidene fluoride (PVDF) membrane, blocked with 10% skim milk at room temperature for 1 hour, then attached with a primary antibody that recognizes and binds to 5-lipoxygenase, and a secondary antibody that recognizes and binds to the primary antibody, and then color-developed with an enhanced chemiluminescent (ECL) solution to measure the expression of 5-LO.

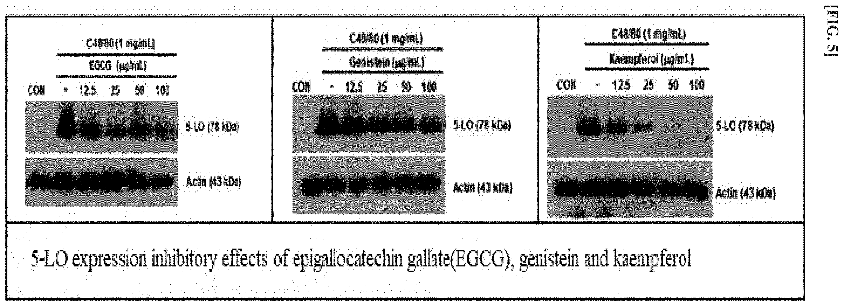

The results are shown in FIG. 5, and it was confirmed that kaempferol effectively inhibits 5-LO expression at the lowest concentration.

2. Inhibition of histamine release

White rat basophil leukemia cells (RBL-2H3) were dispensed into a 24-well plate at a concentration of $1 \times 10^5$ cells/well, and cultured for 24 hours. Samples were added according to the concentrations. After 1 hour, Compound 48/80 (1 mg/ml), which triggers an allergic reaction, was added and cultured for 20 minutes. The well plate was allowed to stand in a refrigerator at 4°C for 10 minutes, and then the medium was collected and the amount of histamine released into the medium was quantified using the Histamine ELISA kit (Neogen). The results are shown in Table 2 below.

As shown in Table 2, it was confirmed that kaempferol inhibited histamine release by more than 50% at a low concentration of 0.2 $\mu$gg/ml.

[Table 2] Inhibitory effect on histamine release in RBL-2H3 cells

|  | Epigallocatechin gallate (EGCG) | Genistein | Kaempferol |
|---|---|---|---|
| histamine release inhibition ($IC_{50}$) | $50.0 \pm 9.80$ $\mu$g/m$\ell$ | > 1000 $\mu$g/m$\ell$ or more. 1000 $\mu$g/m$\ell$ (46%) | $0.20 \pm 0.02$ $\mu$g/m$\ell$ |

Example 4: Measurement of antioxidant activity

1. DPPH radical scavenging ability

100 $\mu\ell$l sample and the same amount of DPPH (2,2-diphenyl-1-picrylhydrazyl, 300 $\mu$ M) solution were mixed in a 96-well plate, and reacted in a dark place for 30 minutes, and the absorbance was measured at 520 nm. The same amount of ethanol was reacted instead of the sample, and the blank value was calculated according to the following Equation.

$$\text{DPPH radical scavenging ability (\%)} = [1- (A\text{-}B) / C] \times 100$$

wherein, A is the absorbance of the sample-added group (DPPH + sample); B is the absorbance of the ethanol-added group (sample + ethanol) instead of DPPH; and C is the absorbance of the ethanol-added group (DPPH + ethanol) instead of the sample.

Sample concentrations when scavenging 50% of DPPH radicals with the $IC_{50}$ value (50% scavenging concentration) of epigallocatechin gallate (EGCG), genistein and kaempferol are shown in Table 3 below. As shown in Table 3, it was confirmed that the $IC_{50}$ value of epigallocatechin gallate (EGCG) was 3.40 $\mu$g/ml, showing the strongest DPPH radical scavenging ability.

[Table 3]

| | Epigallocatechin gallate (EGCG) | Genistein | Kaempferol |
|---|---|---|---|
| DPPH radical scavenging ability (IC$_{50}$) | 3.40 ± 0.13 $\mu$g/m$\ell$ | > 100 $\mu$g/m$\ell$ or more. 5.18% at 100ppm | 11.66 ± 0.30 $\mu$g/m$\ell$ |

2. ABTS radical scavenging ability

ABTS (2,2-azinobis-(3-ethylbenzothiazoline-6-sulphonate) (7 mM in distilled water) and potassium persulfate (2.45 mM in distilled water) were mixed in equal amounts, and allowed to stand in a dark place 12 hours or more to form a ABTS cation. The ABTS mixed solution was diluted with distilled water so that the absorbance value at 734 nm was 0.700±0.050, and then used as a reaction solution. For the measurement, 20 $\mu\ell$ of the sample and 180 $\mu\ell$ of the ABTS reaction solution prepared above were mixed in a 96-well plate, reacted in a dark place for 10 minutes, and then the absorbance was measured at 734 nm. The ABTS radical scavenging ability was calculated according to the following Equation.

$$\text{ABTS radical scavenging ability (\%)} = [1-(A B) / C] \times 100$$

wherein, A is the absorbance of the sample-added group (ABTS + sample); B is the absorbance of the distilled water-added group (sample + distilled water) instead of ABTS; and C is the absorbance of the ethanol-added group (ABTS + ethanol) instead of the sample.

Sample concentrations when scavenging 50% of ABTS radicals with the IC$_{50}$ value (50% scavenging concentration) of epigallocatechin gallate (EGCG), genistein and kaempferol are shown in Table 4 below. As shown in Table 4, it was confirmed that the IC$_{50}$ value of epigallocatechin gallate (EGCG) was 1.65 $\mu$g/ml, showing the strongest ABTS radical scavenging ability.

[Table 4]

| | Epigallocatechin gallate (EGCG) | Genistein | Kaempferol |
|---|---|---|---|
| ABTS radical scavenging ability (IC$_{50}$) | 1.65 ± 0.02 $\mu$g/m$\ell$ | > 100 $\mu$g/m$\ell$ or more. 5.29% at 100ppm | 6.18 ± 0.08 $\mu$g/m$\ell$ |

Example 6: Hair loss amelioration animal test

40 wt.% of kaempferol, 30 wt.% of genistein and 30 wt.% of epigallocatechin gallate were mixed to prepare a composition for ameliorating hair loss (KGE complex) according to the present invention.

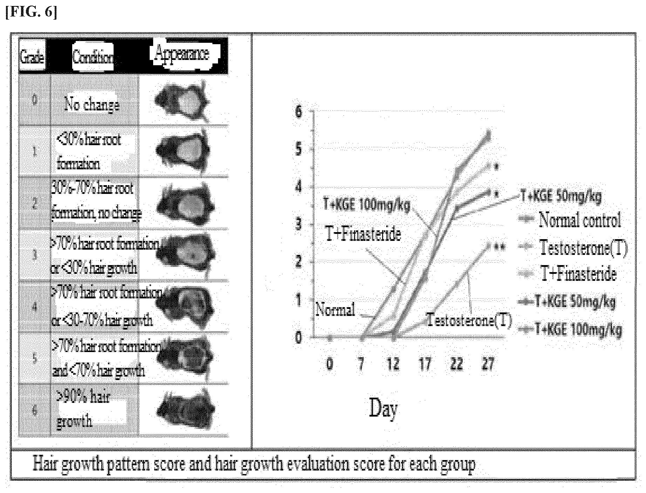

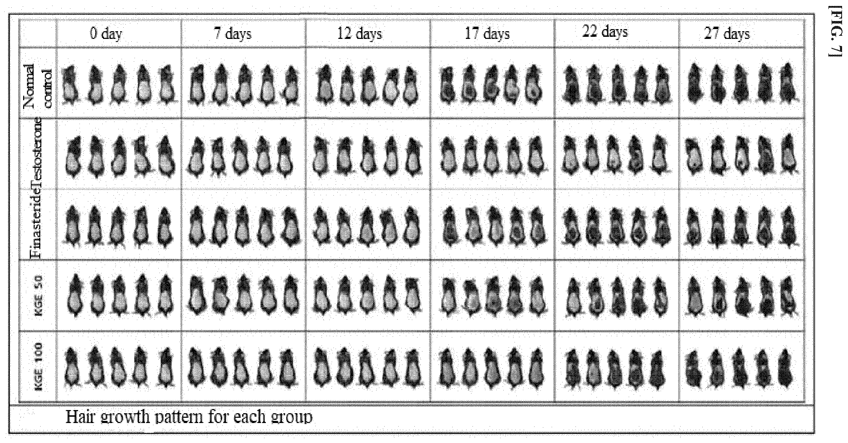

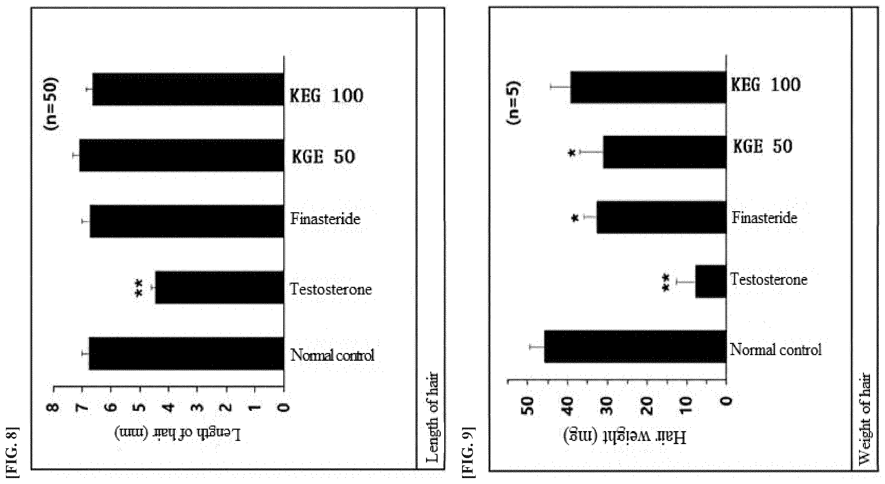

Six-week-old C57BL/6 female mice were obtained from SAMTAKO BIO KOREA CO., LTD., South Korea, and acclimatized for 7 days. The group was then divided into 5 mice per group, and the hair loss animal test was performed. The rearing room was set at a temperature of 22±2°C, a relative humidity of 50±10%, and a lighting cycle of 12 hours, thereby allowing feed and drinking water to freely ingest. The dorsal hair of the mouse was primarily removed using a hair clipper, and then a hair removal cream (Niclean cream available from Ildong Pharmaceutical Co., Ltd., South Korea) was applied thereto for 30 seconds to completely remove hair. Twenty-four hours after hair removal, a corn oil or testosterone propionate (1 mg/ml in corn oil) was subcutaneously injected daily into mice into mice in an amount of 0.1 ml each. The composition for ameliorating hair loss (KGE complex) according to the present invention was mixed and dissolved in a sterilized physiological saline, and 0.1 ml of the solution was orally administered in an amount of 0.1 ml each using a sonde every day. The injection and oral doses for each group are shown in Table 5 below. Just before the start of oral administration (day 0), and on day 7, day 12, day 17, day 22 and day 27 after oral administration, the skin on the back of all mice was photographed with a digital camera to visually evaluate the hair growth rate. The results are shown in FIG. 6 (hair growth pattern score table and hair growth visual evaluation scores for each group) and FIG. 7 (hair growth pattern for each mouse group). After completion of the test (day 27), the grown hair on the central part and the four edge parts was collected with tweezers, and the length was measured using a dissecting microscope and micrometer. The results are shown in FIG. 8 (hair length). The hair remaining on the back removed with a hair clipper, and then the total weight of the grown hair was measured and compared. The results are shown in FIG. 9 (hair weight).

[Table ]

|  | Group | Injection | Oral administration |
|---|---|---|---|
| 1 | Normal control | Corn oil 0.1 mℓ | physiological saline 0.1 mℓ/ mouse |
| 2 | Testosterone propionate | Testosterone propionate (1 mg/mℓ in corn oil) 0.1 mℓ | physiological saline 0.1 mℓ/ mouse |
| 3 | Finasteride | | 1 mg/kg |
| 4 | KGE 50 | | 50 mg/kg |
| 5 | KGE 100 | | 100 mg/kg |

Example 6: Secondary hair loss amelioration animal test

This time, a total of 6 groups were prepared to investigate the synergistic effect of kaempferol and genistein. Each group consisted of 6 mice, and all 6-week-old C57BL/6 female mice were obtained from Samtako BioKorea Co., Ltd. and acclimatized for 7 days. The conditions of the rearing room were set to the same as the previous conditions: a temperature of $22\pm2°C$, a relative humidity of $50\pm10\%$, and a lighting cycle of 12 hours, thereby allowing feed and drinking water to freely ingest. The dorsal hair of the mouse was primarily removed using a hair clipper, and then a hair removal cream (Niclean cream available from Ildong Pharmaceutical Co., Ltd., South Korea) was applied thereto and allowed to stand for 30 seconds. The hair was completely removed while wiping cleanly with a wet tissue. Twenty-four hours after hair removal, testosterone propionate was dissolved at a concentration of 5 mg/ml in corn oil (Group 1 Normal) or corn oil, and the solution was subcutaneously injected into mice in an amount of 0.1 ml each (Groups 2 through 6). Group 1 is a normal group in which only 0.1 ml of corn oil was injected, and 0.1 ml of physiological saline was orally administered using a sonde. Group 2 was subcutaneously injected with testosterone propionate, and this group was also orally ingested with 0.1 ml of physiological saline. All mice belonging to groups 3 to 6 were subcutaneously injected with 0.1 ml of a solution of 5 mg/ml of testosterone propionate in corn oil every day. Group 3 is a group in which finasteride was administered to mice at 1 mg/kg body weight using a sonde every day, Group 4 is a group in which only kaempferol was dissolved in a physiological saline and administered at 100 mg/kg body weight, Group 5 is a group in which only genistein was dissolved in physiological saline and administered at 100 mg/kg body weight, and Group 6 is a group in which in which a mixture of kaempferol and genistein in a weight ratio of 1:1 was administered at 100 mg/kg body weight. Each sample or sample mixture was mixed well with 0.1 ml of sterilized physiological saline at the time of administration. This is summarized in Table 6 below.

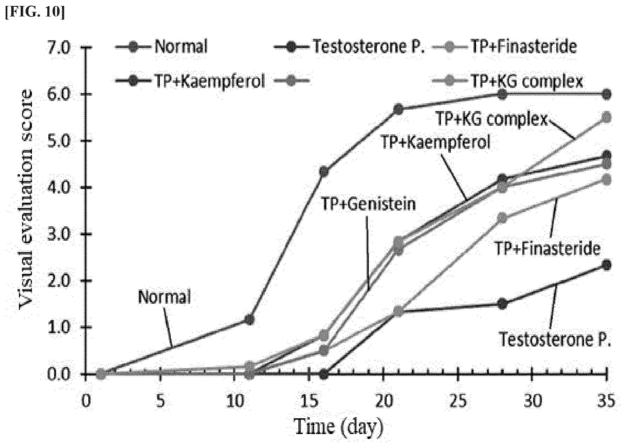

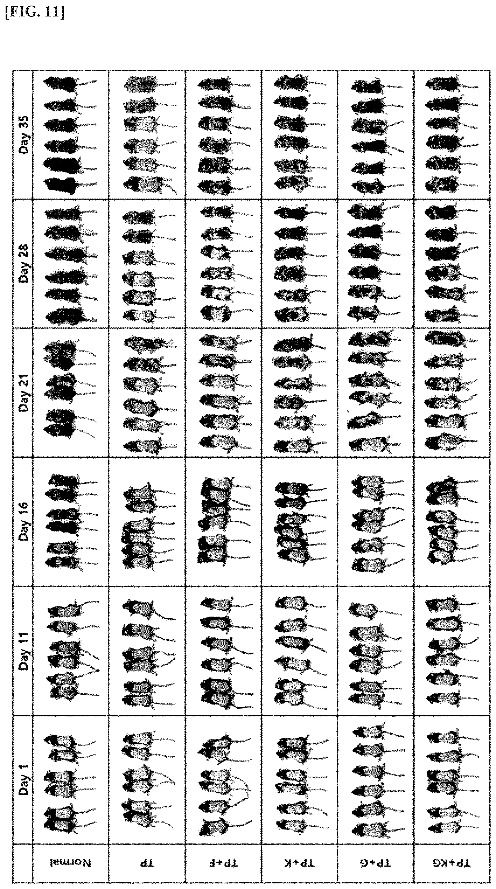

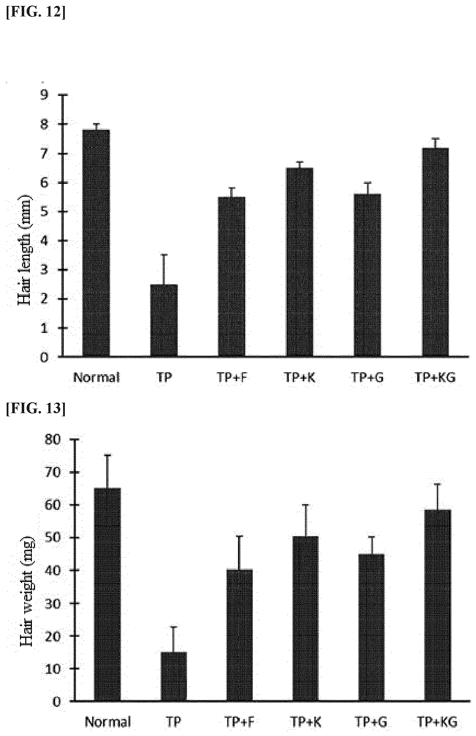

Just before the start of oral administration (day 0), and on day 11, day 16, day 21, day 28, and day 35 after starting oral administration, the backs of all mice during the test were photographed with a digital camera, and hair growth rate and density were scored for each group based on the hair growth pattern score table in FIG. 6. The results are shown in FIG. 10, and FIG. 11 shows the actual mouse hair growth state photographed with a camera arranged by date. After the final observation with taking a picture at the time when 35 days have passed since the start of oral administration, the hair from the central part and the four edges of the area where hair newly grew was collected on the day 35 with tweezers, the length of the hair was measured using a dissecting microscope and a micrometer (FIG. 12), and the remaining hair was cut with a hair clipper, and the total weight of newly grown hair was measured (FIG. 13). Looking at the results, the group in which a mixture of kaempferol and genistein (TP + KG) in a weight ratio of 1:1 was orally administered exhibited more excellent results in terms of hair growth than the group in which kaempferol alone (TP + K) alone or genistein alone (TP + G) was orally administered.

All of these groups showed similar or better results in terms of hair growth than the finasteride oral administration (TP+F) group used in the positive control group.

[Table 6] Injection and oral dosage for each group

|  | Group setting | Injection | Oral administration |
|---|---|---|---|
| 1 | Normal | Corn oil 0.1 mℓ | physiological saline 0.1 mℓ/ mouse |
| 2 | Testosterone propionate (TP) | TP (5 mg/mℓ in corn oil) 0.1 mℓ subcutaneous injection | physiological saline 0.1 mℓ/ mouse |

(continued)

|  | Group setting | Injection | Oral administration |
|---|---|---|---|
| 3 | TP + finasteride (TP+F) | Same as above | 1 mg/kg body weight |
| 4 | TP + kaempferol (TP+K) | Same as above | 100 mg/kg body weight |
| 5 | TP + genistein (TP+G) | Same as above | 100 mg/kg body weight |
| 6 | TP + KG composition (TP+KG) | Same as above | 100 mg/kg body weight |

Example 7: Simple clinical trial on hair loss amelioration

40 wt.% of kaempferol, 30 wt.% of genistein and 30 wt.% of epigallocatechin gallate were mixed to produce a composition for ameliorating hair loss (KGE complex) according to the present invention.

A brief clinical trial was conducted on voluntary participants (6 females, 9 males) between the ages of 34 and 67 who complained of hair loss symptoms.

80 mg of the composition for ameliorating hair loss (KGE complex) was filled into capsules, and then provided to the subjects, and they were instructed to ingest 1 capsule twice a day (morning and evening) for 8 weeks. After confirming the health condition of the subject's scalp before the start of the test, the effect of ameliorating hair loss was evaluated every 4 weeks and 8 weeks through visual inspection and hair skin diagnosis. The number of hairs (pcs/cm$^2$) and hair thickness (mm) were measured and evaluated using a hair & skin diagnostic device (AramHuvis Co., Ltd., South Korea) by the following methods:

1) Evaluation of hair density

The number of hairs per unit area (1 cm$^2$) was calculated and indicated by multiplying the number of hairs in the corresponding area (0.25 cm$^2$) measured using a hair & skin diagnostic device by 4.

2) Hair thickness

The thickness of 10 hairs was measured using a hair & skin diagnostic device, and the average value was expressed as the hair thickness (mm).

In addition, in order to ascertain whether or not the subjects themselves felt amelioration of hair loss, a questionnaire survey was conducted by defining the question items as shown in Table 7, and the results are also shown in Table 7 below.

As shown in Table 7, as a result of the questionnaire survey, 2 subjects selected very good (+3 points) in the item [hair on the top of head became abundant], and 9 subjects selected "improved" (+2 points), resulting in a total score of 25 points. In the item [the number of hairs falling out was decreased], 3 subjects selected "very improved" (+3 points), 5 subjects selected "improved" (+2 points), and 5 subjects selected " slightly improved" (+1 point), resulting in a total score of 24 points. As a result, it was confirmed that most of the subjects who participated in the test felt the effect of ameliorating hair loss symptoms.

[Table 7]

| No. | Question item | Number of subjects selected for applicable scores | | | | | | | |
|---|---|---|---|---|---|---|---|---|---|
| | | Very improved (+3 point) | Improved (+2 point) | slightly improved (+1 point) | No change (0 point) | Slightly worsened (-1 point) | Worsened (-2 point) | Very worsened (-3 point) | |
| 1 | Hair on the top of head became abundant | 2 | 9 | 1 | 3 | | | | 25 |
| 2 | Number of hairs falling out decreased | 3 | 5 | 5 | 2 | | | | 24 |
| 3 | Front hair line was improved | 2 | 6 | 4 | 3 | | | | 22 |

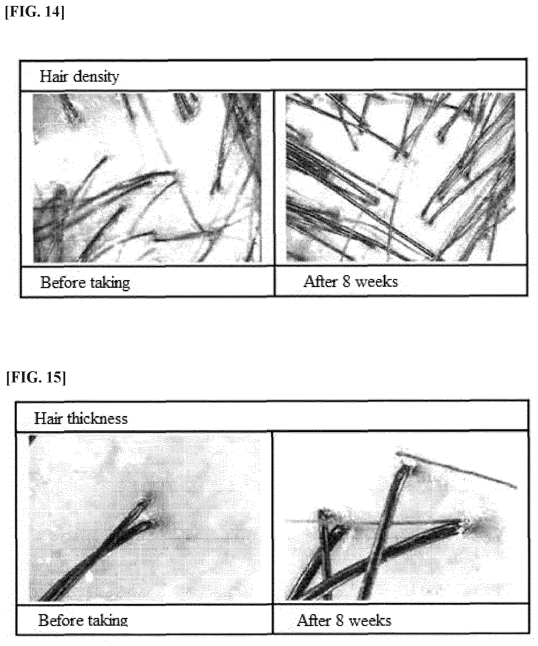

After a simple clinical trial for 8 weeks, the results of changes in the number of hairs and hair thickness are shown in Table 7, FIGS. 14 and 15.

[Table 8]

| No. | Gender/Age | number of hairs (hair/cm$^2$) | | | Increase/ decrease in number of hairs (%) | hair thickness (mm) | | | Increase/ decrease in hair thickness (%) |
|---|---|---|---|---|---|---|---|---|---|
| | | Before taking | After 4 weeks | After 8 weeks | | Before taking | After 4 weeks | After 8 weeks | |
| 1 | Male/47 | 120 | 125 | 125 | 4.2 | 0.059 | 0.062 | 0.077 | 30.5 |
| 2 | Female/54 | 110 | 120 | 130 | 18.2 | 0.056 | 0.066 | 0.080 | 42.9 |
| 3 | Female/34 | 119 | 135 | 146 | 22.7 | 0.055 | 0.069 | 0.077 | 40.0 |
| 4 | Male/49 | 99 | 100 | 110 | 11.1 | 0.063 | 0.075 | 0.075 | 19.0 |
| 5 | Female/44 | 116 | 140 | 152 | 31.0 | 0.063 | 0.088 | 0.083 | 31.7 |
| 6 | Male/65 | 101 | 118 | 120 | 18.8 | 0.062 | 0.070 | 0.080 | 29.0 |
| 7 | Female/35 | 130 | 140 | 158 | 21.5 | 0.054 | 0.064 | 0.066 | 22.2 |
| 8 | Male/67 | 90 | 108 | 110 | 22.2 | 0.050 | 0.066 | 0.070 | 40.0 |
| 9 | Female/43 | 78 | 76 | 78 | 0.0 | 0.068 | 0.066 | 0.068 | 0.0 |
| 10 | Male/55 | 88 | 105 | 109 | 23.9 | 0.066 | 0.000 | 0.078 | 18.2 |
| 11 | Male/43 | 115 | 120 | 130 | 13.0 | 0.063 | 0.980 | 0.1247 | 96.8 |
| 12 | Male/57 | 70 | 88 | 90 | 28.6 | 0.082 | 0.080 | 0.100 | 22.0 |
| 13 | Male/45 | 68 | 80 | 98 | 44.1 | 0.062 | 0.066 | 0.070 | 12.9 |
| 14 | Female/59 | 110 | 115 | 115 | 4.5 | 0.090 | 0.110 | 0.110 | 22.2 |
| 15 | Male/39 | 73 | 103 | 110 | 50.7 | 0.066 | 0.070 | 0.076 | 15.2 |
| Increase/decrease of average value(%) | | | | | 21.0 | | | | 29.5 |

It was confirmed that the number of hairs of the subjects increased by an average of 21.0% and the hair thickness increased by an average of 29.5%. In addition, it was confirmed that the average number of hairs increased by 10% or more in all 12 of the 15 subjects, excluding 3, and the thickness of hairs in all 14 subjects increased by 10% or more.

Although the present invention has been described in detail only with respect to the embodiments described above, it will be apparent to those skilled in the art that various variations and modifications can be made to the invention within the scope of the technical idea of the present invention, and it goes without saying that such variations and modifications naturally fall within the scope of the appended claims.

**Claims**

1. A pharmaceutical composition for alleviating or treating hair loss comprising genistein and kaempferol as active ingredients.

2. The pharmaceutical composition for alleviating or treating hair loss according to claim 1, which contains genistein in an amount ranging from 1 to 95% by weight and kaempferol in an amount ranging from 5 to 95%.

3. The pharmaceutical composition for alleviating or treating hair loss according to claim 1, further comprising epigallocatechin gallate (EGCG).

4. The pharmaceutical composition for alleviating or treating hair loss according to claim 3, which contains 2 to 90% by weight of genistein, 10 to 90% by weight of kaempferol and the balance of epigallocatechin gallate.

5. The pharmaceutical composition for alleviating or treating hair loss according to claim 1, wherein genistein and kaempferol are extracted and concentrated synthetically or naturally.

6. The pharmaceutical composition for alleviating or treating hair loss according to claim 5, wherein genistein is extracted and concentrated from lupine, fava beans, soy beans, kudzu root or Sophora japonica (pagoda tree) fruits.

7. The pharmaceutical composition for alleviating or treating hair loss according to claim 1, further comprising a yeast extract,

8. The pharmaceutical composition for alleviating or treating hair loss according to claim 7, wherein the yeast extract is contained in an amount ranging from 10 to 50 parts by weight based on 100 parts by weight of the pharmaceutical composition for alleviating or treating hair loss.

9. A pharmaceutical product (quasi-drug) for alleviating or treating hair loss, comprising the pharmaceutical composition for alleviating or treating hair loss according to any one of claims 1 to 8.

10. The pharmaceutical product (quasi-drug) for alleviating or treating hair loss according to claim 9, wherein the pharmaceutical product (quasi-drug) for alleviating or treating hair loss is disinfection cleaners, shower foams, detergent soaps, hand washes, gels, hair packs, shampoos, scalp toners, scalp ampoules, suspensions, emulsions, pastes, ointments, oils or shower filter fillers.

11. A cosmetic composition for alleviating or treating hair loss, comprising the pharmaceutical composition for alleviating or treating hair loss according to any one of claims 1 to 8.

12. The cosmetic composition for alleviating or treating hair loss according to claim 11, wherein the cosmetic composition for alleviating or treating hair loss is formulated into solutions, suspensions, emulsions, pastes, gels, creams, lotions, powders, shampoos, scalp toners, scalp ampoules, soaps, surfactant-containing cleansers, oils, powder foundations, emulsion foundations, wax foundations and sprays.

13. A functional food composition for alleviating or treating hair loss, comprising the pharmaceutical composition for alleviating or treating hair loss according to any one of claims 1 to 8.

14. A composition of any one of claims 1 to 6, using a mechanism by which when dermal papilla cells are exposed to androgen hormones for a long period of time, IL-6 or TNF-$\alpha$, which is a pro-inflammatory factor, is produced and secreted to cause inflammation in the hair follicle tissue, finally entirely debilitating the cells and tissues that make up the hair follicle, and inhibiting induction of hair loss.

15. A composition of any one of claims 1 to 6, using a mechanism that induces hair loss by long-term exposure to IL-6 or TNF-$\alpha$ produced and secreted by macrophages scattered in hair follicles and tissues surrounding the hair follicles.

16. A composition of any one of claims 1 to 6, using a mechanism that inhibits the production and secretion of histamine, an allergy-inducing substance produced by mast cells scattered in hair follicles and in the periphery of hair follicles, thereby causing hair loss.

17. A composition of any one of claims 1 to 6, which inhibits the expression of 5-lipoxygenase involved in the synthesis of leukotriene, an allergenic substance produced by mast cells scattered in hair follicles and in the periphery of hair follicles to thereby ameliorate or treat hair loss.

18. A composition of any one of claims 1 to 6, which inhibits the expression of 5$\alpha$-reductase that catalyzes the conversion of testosterone to dihydrotestosterone, and finally lowers the concentration of dihydrotestosterone in blood to thereby ameliorate or treat hair loss.

19. A composition of any one of claims 1 to 6, which inhibits the production and secretion of pro-inflammatory cytokines such as IL-6 or TNF-$\alpha$ that cause inflammatory reactions in the scalp or hair follicles and tissues around hair follicles due to extreme stress, external pollutants, environmental hormones, and the like to thereby alleviate or treat alopecia areata.

20. A composition of any one of claims 1 to 6, which prevents the production and secretion of pro-inflammatory cytokines such as IL-6 or TNF-$\alpha$ caused by high blood testosterone and high blood levels of 5a-reductase-catalyzed dihydrotestosterone.

21. A composition of any one of claims 1 to 6, which alleviates inflammation of hair follicles, peripheral tissues and cells by using 5 to 9,500 mg/day of kaempferol as a sole active ingredient to thereby treat or alleviate hair loss.

22. A composition which alleviates inflammation of hair follicles, peripheral tissues and cells by using 5 to 950 mg/day of genistein as a sole active ingredient to thereby treat or alleviate hair loss.

[FIG. 1]

iNOS and COX-2 expression inhibitory effects of epigallocatechin gallate., genistein and kaempferol in RAW 264.7 cells

[FIG. 2]

[FIG. 3]

[FIG. 4]

5α-reductase type II expression inhibitory effects of epigallocatechin gallate (EGCG), genistein and kaempferol

[FIG. 5]

5-LO expression inhibitory effects of epigallocatechin gallate(EGCG), genistein and kaempferol

EP 4 324 466 A1

[FIG. 6]

Hair growth pattern score and hair growth evaluation score for each group

25

[FIG. 7]

Hair growth pattern for each group

[FIG. 8]

Length of hair

[FIG. 9]

Weight of hair

[FIG. 10]

[FIG. 11]

[FIG. 12]

[FIG. 13]

[FIG. 14]

| Hair density | |
|---|---|
| Before taking | After 8 weeks |

[FIG. 15]

| Hair thickness | |
|---|---|
| Before taking | After 8 weeks |

## INTERNATIONAL SEARCH REPORT

| International application No. |
|---|
| **PCT/KR2022/003976** |

| | |
|---|---|
| **A.** | **CLASSIFICATION OF SUBJECT MATTER** |
| | **A61K 31/7048**(2006.01)i; **A61K 31/352**(2006.01)i; **A61K 31/353**(2006.01)i; **A61K 36/48**(2006.01)i; **A61K 36/488**(2006.01)i; **A61K 36/489**(2006.01)i; **A61K 36/06**(2006.01)i; **A61P 17/14**(2006.01)i; **A61K 8/49**(2006.01)i; **A23L 33/105**(2016.01)i |
| | According to International Patent Classification (IPC) or to both national classification and IPC |

| | |
|---|---|
| **B.** | **FIELDS SEARCHED** |
| | Minimum documentation searched (classification system followed by classification symbols) |
| | A61K 31/7048(2006.01); A61K 36/13(2006.01); A61K 8/97(2006.01); A61K 8/9761(2017.01); A61K 8/99(2006.01) |

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 제니스테인(genistein), 켐페롤(kaempferol), 탈모(alopecia), 에피갈로카테킨 갈레이트(epigallocatechin gallate), 효모 추출물(yeast extract), 염증(inflammatory), 모낭(hair follicle)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| X | US 2003-0105030 A1 (LIAO, S. et al.) 05 June 2003 (2003-06-05)<br>See claims 1, 3, 9-12, 20 and 56; paragraphs [0133], [0190] and [0192]; and table 3. | 1-6,9-13,22 |
| Y | | 7-8 |
| Y | KR 10-2017-0074786 A (PARK, Min Sun) 30 June 2017 (2017-06-30)<br>See claims 1 and 8; and paragraphs [0023]-[0025]. | 7-8 |
| X | KR 10-2020-0064309 A (AMOREPACIFIC CORPORATION) 08 June 2020 (2020-06-08)<br>See claims 1, 5-6, 10-11 and 14; and table 1. | 22 |
| A | US 2007-0207228 A1 (GIULIANI, G. et al.) 06 September 2007 (2007-09-06)<br>See entire document. | 1-13,22 |
| A | US 2003-0229030 A1 (THEOHARIDES, Theoharis C.) 11 December 2003 (2003-12-11)<br>See entire document. | 1-13,22 |

| | |
|---|---|
| ☐ Further documents are listed in the continuation of Box C. | ☑ See patent family annex. |

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **30 June 2022** | **30 June 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2022/003976** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☑ Claims Nos.: **14-21**
because they are dependent claims and are not drafted in accordance with the second and third sentences of Rule 6.4(a).

Form PCT/ISA/210 (continuation of first sheet) (July 2019)

**INTERNATIONAL SEARCH REPORT**
**Information on patent family members**

International application No.

**PCT/KR2022/003976**

| Patent document cited in search report | | | Publication date (day/month/year) | Patent family member(s) | | | Publication date (day/month/year) |
|---|---|---|---|---|---|---|---|
| US | 2003-0105030 | A1 | 05 June 2003 | US | 6696484 | B2 | 24 February 2004 |
| KR | 10-2017-0074786 | A | 30 June 2017 | KR | 10-1888419 | B1 | 14 August 2018 |
| KR | 10-2020-0064309 | A | 08 June 2020 | CN | 113164365 | A | 23 July 2021 |
| | | | | JP | 2022-510097 | A | 26 January 2022 |
| | | | | WO | 2020-111621 | A1 | 04 June 2020 |
| US | 2007-0207228 | A1 | 06 September 2007 | AU | 2005-219032 | A1 | 15 September 2005 |
| | | | | AU | 2005-219032 | B2 | 26 November 2009 |
| | | | | CA | 2558150 | A1 | 15 September 2005 |
| | | | | CN | 1942168 | A | 04 April 2007 |
| | | | | EP | 1720515 | A1 | 15 November 2006 |
| | | | | JP | 2007-526297 | A | 13 September 2007 |
| | | | | WO | 2005-084621 | A1 | 15 September 2005 |
| US | 2003-0229030 | A1 | 11 December 2003 | | None | | |

Form PCT/ISA/210 (patent family annex) (July 2019)